# EUROPÄISCHE PATENTANMELDUNG

(11) **EP 4 763 115 A1**
(43) Veröffentlichungstag der Anmeldung: **24.06.2026**
(21) Anmeldenummer: 25220640.4
(22) Anmeldetag: 04.12.2025
(51) Int. Cl.: A61B 17/80

(54) **OSTEOSYNTHESEPLATTE**

(30) Priorität: 19.12.2024 DE 102024138940
(71) Anmelder: Karl Leibinger Asset Management GmbH & Co. KG, 78570 Mühlheim an der Donau (DE)
(72) Erfinder: Leibinger, Ralf, 78570 Mühlheim an der Donau (DE); Reinauer, Frank, 78576 Emmingen-Liptingen (DE); Aberle, Steffen, 78126 Königsfeld (DE)
(74) Vertreter: Schwamberger, Martin

(57) **Zusammenfassung**

1. Osteosyntheseplatte (OP; OP'; OP"; OP‴), welche für eine Fixierung von Knochenfragmenten (K1, K2) zur Wiederherstellung eines Knochens (K), insbesondere einer Mandibula, vorgesehen ist, umfassend einen Plattenkörper (P; P') mit einer Oberseite (OS) und einer Unterseite, auf welcher der Plattenkörper (P; P') an den Knochenfragmenten (K1, K2) aufzulegen ist, wobei der Plattenkörper (P; P') mehrere Befestigungsabschnitte (BA1, BA2; BA1', BA2', BA3') aufweist, an welchen auf der Unterseite Befestigungen der Osteosyntheseplatte (OP; OP'; OP"; OP‴) seitens der Knochenfragmente (K1, K2) vorzunehmen sind und denen dazu jeweils mindestens ein Befestigungspunkt (BP1, BP2; BP1', BP2', BP3') zugeordnet ist, wobei der mindestens eine Befestigungspunkt (BP2; BP2', BP3') bei zumindest einem der Befestigungsabschnitte (BA1, BA2; BA1', BA2', BA3') an einem Inselabschnitt (IA) ausgestaltet ist, welcher mit dem zugehörigen Befestigungsabschnitt (BA2; BA2', BA3') des Plattenkörpers (P; P') ausschließlich über Federsegmente (FS1, FS2) verbunden ist, welche eine Relativverschiebung des jeweiligen Befestigungsabschnitts (BA2; BA2', BA3') und des jeweiligen Inselabschnitts (IA) zueinander elastisch federnd zulassen, wobei die Federsegmente (FS1, FS2) den ihnen zugeordneten Befestigungspunkt (BP2, BP2', BP3') asymmetrisch umgeben.

## Beschreibung

Die Erfindung betrifft eine Osteosyntheseplatte, welche für eine Fixierung von Knochenfragmenten zur Wiederherstellung eines Knochens, insbesondere einer Mandibula, vorgesehen ist, umfassend einen Plattenkörper mit einer Oberseite und einer Unterseite, auf welcher der Plattenkörper an den Knochenfragmenten aufzulegen ist. Der Plattenkörper weist zumindest einen Brückenabschnitt zum Überbrücken eines zugehörigen, zwischen den Knochenfragmenten verlaufenden Spalts auf, wobei der Plattenkörper mit beidseitig des zumindest einen Brückenabschnitts liegenden Befestigungsabschnitten ausgestattet ist. Die Osteosyntheseplatte ist an ihrer Unterseite mittels den Befestigungsabschnitten an den Knochenfragmenten zu befestigen. Dazu ist jedem Befestigungsabschnitt mindestens ein Befestigungspunkt zugeordnet, wobei der mindestens eine Befestigungspunkt bei zumindest einem der Befestigungsabschnitte an jeweils einem Inselabschnitt ausgestaltet ist. Der Inselabschnitt ist mit dem jeweils zugehörigen Befestigungsabschnitt ausschließlich über Federsegmente verbunden, welche eine Relativverschiebung des jeweiligen Befestigungsabschnitts und des jeweiligen Inselabschnitts elastisch federnd zueinander zulassen.

Bei der Osteosynthese werden im Rahmen eines operativen Eingriffs zwei oder mehr Knochenfragmente miteinander verbunden, um ein Zusammenwachsen der Knochenfragmente und damit eine Wiederherstellung des jeweiligen Knochens zu erreichen. Ziel der Osteosynthese ist dabei eine Stabilisierung der Knochenfragmente zueinander, wobei diese Stabilisierung dabei in einer korrekten Stellung und somit ggf. unter Korrektur von Fehlstellungen erfolgen soll. Neben einer Fixierung über Draht oder Schrauben kommen je nach Anwendungsbereich auch Osteosyntheseplatten zur Anwendung, wobei die jeweilige Osteosyntheseplatte dabei im Bereich eines jeweiligen Spalts zwischen den Knochenfragmenten auf den jeweiligen Knochen aufgelegt und jeweils an den miteinander zu verbindenden Knochenfragmenten befestigt wird.

Neben einer Stabilisierung von Knochenfragmenten nach einer Knochenfraktur kommen Osteosyntheseplatten teilweise auch dann zur Anwendung, wenn bei einem Knochen ein an anderer Stelle reseziertes Knochensegment an einer vorhandenen Fehlstelle des Knochens einzusetzen und für eine Wiederherstellung des Knochens zu stabilisieren ist. So kann eine derartige Fehlstelle beispielsweise aufgrund einer notwendigen Entfernung eines Knochenteils des Knochens nach einer Tumorerkrankung entstanden sein.

Hinsichtlich eines Heilungsprozesses des jeweiligen Knochens hat es sich als vorteilhaft erwiesen, wenn die Knochenfragmente des wiederherzustellenden Knochens nicht starr über die jeweilige Osteosyntheseplatte miteinander verbunden, sondern Mikrobewegungen der Knochenfragmente zueinander ermöglicht werden. Um diese Mikrobewegungen zu ermöglichen, werden die Befestigungspunkte auf Seiten zumindest eines der zu verbindenden Knochenfragmente über Federsegmente angebunden.

Die WO 2011/163387 A2 beschreibt eine Knochenbruchfixierungsplatte mit einer Außenfläche und einer dem Knochen zugewandten Fläche. Die Knochenplatte weist Schlitze auf, die sich durch die Platte von der Außenfläche zur dem Knochen zugewandten Fläche erstrecken. Die Schlitze umschreiben zumindest teilweise einen Umfang eines oder mehrerer Aufnahmelöcher und gehen nicht durch eine Längskante der Knochenplatte hindurch. Die Schlitze bilden ein Federelement, welche das Aufnahmeloch zumindest teilweise umgibt und eine axiale Verschiebung der Knochenplatte relativ zu dem einen oder den mehreren Aufnahmelöchern innerhalb einer Ebene ermöglicht, die parallel zu einer oberen oder unteren Fläche der Knochenplatte ist, aber eine Bewegung der Knochenplatte relativ zu dem einen oder den mehreren Aufnahmelöchern in einer Richtung verhindert, die senkrecht zur Oberseite der Knochenplatte ist.

Ausgehend vom vorstehend beschriebenen Stand der Technik ist es nun die Aufgabe der vorliegenden Erfindung eine Osteosyntheseplatte zu schaffen, bei welcher eine möglichst variable Anordnung von über Federsegmente relativ verschiebbaren Befestigungspunkten verwirklicht werden kann.

Diese Aufgabe wird ausgehend vom Oberbegriff des Anspruchs 1 in Verbindung mit dessen kennzeichnenden Merkmalen gelöst. Die hierauf folgenden, abhängigen Ansprüche geben jeweils vorteilhafte Weiterbildungen der Erfindung wieder. Weitere vorteilhafte Ausgestaltungen ergeben sich aus der Beschreibung sowie aus den Figuren.

Gemäß der Erfindung umfasst eine Osteosyntheseplatte, welche für eine Fixierung von Knochenfragmenten zur Wiederherstellung eines Knochens vorgesehen ist, einen Plattenkörper mit einer Oberseite und einer Unterseite, auf welcher der Plattenkörper an den Knochenfragmenten aufzulegen ist. Der Plattenkörper weist mehrere Befestigungsabschnitte auf, an welchen auf der Unterseite Befestigungen der Osteosyntheseplatte seitens der Knochenfragmente vorzunehmen sind und denen dazu jeweils mindestens ein Befestigungspunkt zugeordnet ist. Der mindestens eine Befestigungspunkt ist bei zumindest einem der Befestigungsabschnitte an einem Inselabschnitt ausgestaltet, welcher mit dem zugehörigen Befestigungsabschnitt des Plattenkörpers ausschließlich über Federsegmente verbunden ist, welche eine Relativverschiebung des jeweiligen Befestigungsabschnitts und des jeweiligen Inselabschnitts zueinander elastisch federnd zulassen.

Die erfindungsgemäße Osteosyntheseplatte ist für eine Fixierung von Knochenfragmenten zur Wiederherstellung eines Knochens vorgesehen. Diese Wiederherstellung des Knochens kann dabei im Rahmen der Erfindung zum einen derartig vorliegen, dass über die erfindungsgemäße Osteosyntheseplatte Knochenfragmente aneinander fixiert werden, die sich aufgrund einer Knochenfraktur des Knochens gebildet haben. Somit werden in diesem Fall die dem wiederherzustellenden Knochen zugeordneten Knochenfragmente über die Osteosyntheseplatte zueinander fixiert und stabilisiert. Zum anderen wird im Sinne der Erfindung als Wiederherstellung eines Knochens aber auch angesehen, dass eine Fehlstelle eines Knochens mit einem Knochensegment geschlossen wird, welches zu diesem Zweck bei einem anderen Knochen reseziert worden ist. Dementsprechend wird der jeweilige Knochen in diesem Fall aus eigenen Knochenfragmenten und einem anderen Knochen zugehörigen Knochenfragment wiederhergestellt. Die erfindungsgemäße Osteosyntheseplatte dient in diesem Fall dann der Fixierung des die Fehlstelle schließenden, resezierten Knochensegments mit mindestens einem der beidseitig liegenden Knochensegmente. Ganz besonders bevorzugt ist die Osteosyntheseplatte für die Verwendung im Bereich des Unterkieferknochens (Mandibula) vorgesehen, wobei hierbei dann entweder eine Wiederherstellung im Rahmen einer Knochenfraktur oder im Rahmen des Schließens einer Knochenfehlstelle verwirklicht sein kann.

Die Osteosyntheseplatte ist mit einem Plattenkörper ausgestattet, welcher bevorzugt länglich ausgeführt ist, d.h. sich insbesondere zumindest überwiegend in einer Längsrichtung erstreckt. Dabei kann sich der Plattenkörper entlang einer in diese Längsrichtung verlaufenden Längsachse ausdehnen oder entlang einer in diese Längsrichtung orientierten Leitkurve verlaufend ausgestaltet sein.

Der Plattenkörper verfügt über eine Oberseite und eine Unterseite, welche bei dem Plattenkörper insbesondere einander abgewandt orientiert sind. Auf der Unterseite des Plattenkörpers findet dabei bei Verwendung der erfindungsgemäßen Osteosyntheseplatte das Auflegen des Plattenkörpers auf den Knochenfragmenten statt, wobei bei diesem Auflegen zumindest ein Spalt überbrückt wird, welcher zwischen den zu fixierenden Knochenfragmenten verläuft. Zudem wird auf der Unterseite des Plattenkörpers bei Verwendung der Osteosyntheseplatte eine Befestigung seitens der zu fixierenden Knochenfragmente vorgenommen, wozu der Plattenkörper der Osteosyntheseplatte mit mehreren Befestigungsabschnitten ausgestattet ist. Jedem der Befestigungsabschnitte ist hierbei mindestens ein Befestigungspunkt zugeordnet, wobei der jeweilige Befestigungspunkt dabei bevorzugt durch jeweils eine Durchgangsöffnung gebildet ist, durch welche eine Knochenschraube hindurchgeführt werden kann. Mittels der Knochenschraube wird dabei die Befestigung an dem jeweiligen Knochenfragment vorgenommen.

Bei zumindest einem der Befestigungsabschnitte des Plattenkörpers ist der mindestens eine vorgesehene Befestigungspunkt an einem Inselabschnitt ausgestaltet, welcher mit dem zugeordneten Befestigungsabschnitt ausschließlich über Federsegmente verbunden ist, welche den jeweiligen Inselabschnitt mit dem zugeordneten Befestigungsabschnitt koppeln. Diese Federsegmente lassen eine Relativverschiebung des Inselabschnitts zu dem zugehörigen Befestigungsabschnitt elastisch federnd zu, worunter im Sinne der Erfindung insbesondere zu verstehen ist, dass sich das Federsegment bei einer Relativverschiebung, welche der Inselabschnitt und der zugehörige Befestigungsabschnitt zueinander bei Belastung vornehmen, elastisch verformt und sich bei Entlastung unter Rückbewegung in eine Ausgangsstellung wieder zurückverformt. Bevorzugt ist das Federsegment als zumindest weitestgehend linear verlaufender Federschenkel ausgestaltet.

Bevorzugt lassen die Federsegmente die Relativverschiebung zwischen Inselabschnitt und Befestigungsabschnitt nur weitestgehend parallel zu der Ober- und der Unterseite und/oder in Längsrichtung zu. Denn da in dieser Richtung aufgrund des Verlaufs des Plattenkörpers auch die Überbrückung des jeweiligen Spalts zwischen den Knochenfragmenten stattfindet, haben in diese Richtung orientierte Relativverschiebungen Änderungen des Spaltmaßes zur Folge, was sich hinsichtlich des Heilungsprozesses des wiederherzustellenden Knochens als vorteilhaft erwiesen hat.

Besonders bevorzugt steht der jeweilige Inselabschnitt auf der Unterseite des Plattenkörpers gegenüber dem zugehörigen Befestigungsabschnitt vor, wodurch bei Befestigung der Osteosyntheseplatte am des jeweiligen Knochenfragment eine Auflage an dem jeweiligen Inselabschnitt bei gleichzeitigem Freiraum des Knochenfragments zu dem zugehörigen Befestigungsabschnitt stattfindet. Hierdurch kann sichergestellt werden, dass die Relativverschiebungen zwischen Inselabschnitt und Befestigungsabschnitt nicht dadurch reibungsbedingt behindert werden, dass auch der Befestigungsabschnitt auf dem ebenfalls relativ zu dem Befestigungsabschnitt zu verschiebenden Knochenfragment aufliegt.

Die Osteosyntheseplatte wird vorzugsweise mittels eines additiven Fertigungsverfahrens hergestellt, bevorzugt mittels eines 3D-Druckverfahrens. Die Osteosyntheseplatte besteht vorzugsweise aus Titan, einer Titanlegierung, aus Edelstahl oder aus einem geeigneten Polymer wie beispielsweise Polyetheretherketon (PEEK).

Die Erfindung umfasst nun die technische Lehre, dass die Federsegmente den ihnen zugeordneten Befestigungspunkt asymmetrisch umgeben.

Eine derartige Ausgestaltung einer Osteosyntheseplatte hat dabei den Vorteil, dass durch die asymmetrischen Verläufe der Federsegmente eine kompakte Ausgestaltung des Inselabschnitts an dem zugehörigen Befestigungsabschnitt möglich ist. So kann bei einem Inselabschnitt, welcher bei dem zugehörigen Befestigungsabschnitt an einem Ende des Plattenkörpers angeordnet ist, das auf Seiten dieses Endes liegende Federsegment abweichend von dem gegenüberliegenden Federsegment gestaltet werden, um den Inselabschnitt möglichst dicht an dem Ende des Plattenkörpers anordnen zu können. Ist der Befestigungsabschnitt mit mehreren an Inselabschnitten ausgebildeten Befestigungspunkten versehen, so ermöglichen die asymmetrischen Verläufe der jeweils Federsegmente eine dichtere Anordnung dieser Inselabschnitte. Somit ermöglicht der erfindungsgemäße Aufbau eine höhere Variabilität bei der Anordnung der Befestigungspunkte, sodass die Anordnung bestmöglich an die Anatomie angepasst werden kann.

Entsprechend einer möglichen Ausführungsform der Erfindung ist der Inselabschnitt über genau zwei Federsegmente mit dem jeweiligen Befestigungsabschnitt verbunden. In diesem Fall koppeln also genau zwei Federsegmente den jeweiligen Inselabschnitt mit dem zugehörigen Befestigungsabschnitt. Im Rahmen der Erfindung könnten jedoch auch mehr als zwei Federsegmente zur Verbindung des jeweiligen Inselabschnitt mit dem zugehörigen Befestigungsabschnitt vorgesehen sein.

Gemäß einer weiteren Ausgestaltungsmöglichkeit der Erfindung sind der Inselabschnitt und die zugeordneten Federsegmente durch an dem jeweiligen Befestigungsabschnitt ausgestaltete Durchbrüche gebildet. In vorteilhafter Weise lässt sich hierdurch eine einteilige Ausgestaltung des Plattenkörpers verwirklichen, indem die Definition des Inselabschnitts und der Federsegmente durch Einbringen der Durchbrüche in den Plattenkörper an dem jeweiligen Befestigungsabschnitt vorgenommen wird. Wird der Plattenkörper der Osteosyntheseplatte dabei mittels eines additiven Fertigungsverfahrens hergestellt, so können auch die Durchbrüche bei dieser additiven Herstellung ausgestaltet worden sein. Alternativ dazu können die Durchbrüche aber auch abrasiv oder spanabhebend definiert sein.

Die Durchbrüche können U-förmig ausgestaltet sein, wobei die U-förmigen Durchbrüche mit ihren Öffnungen einander zugewandt und ineinander geschachtelt ausgestaltet sind. Dadurch können der jeweilige Inselabschnitt und die zugehörigen Federsegmente besonders platzsparend gestaltet werden, sodass die Variabilität der Anordnung der Befestigungspunkte weiter verbessert wird.

Es ist eine weitere mögliche Ausführungsform der Erfindung, dass ein erstes der Federsegmente orthogonal zu einer Längsrichtung des Befestigungsabschnitts orientiert ist. Durch diesen Verlauf des Federsegments kann der zugehörige Inselabschnitt nahe zu einem Ende des zugehörigen Befestigungsabschnitts oder angrenzend zu einem weiteren Inselabschnitt des zugehörigen Befestigungsabschnitts angeordnet werden.

Entsprechend einer weiteren Ausgestaltungsmöglichkeit der Erfindung sind dem jeweiligen Befestigungsabschnitt mehrere Befestigungspunkte zugeordnet, wobei jeder der Befestigungspunkte an jeweils einem Inselabschnitt mit asymmetrisch um den jeweiligen Befestigungspunkt angeordneten Federsegmenten ausgestaltet sind. Hierdurch können bei diesem Befestigungsabschnitt die mehreren Befestigungspunkte kompakt zueinander angeordnet werden, sodass die Variabilität der Anordnung der Befestigungspunkte weiter verbessert wird.

Vorzugsweise ist ein zweites der Federsegmente spitzwinkelig zu einer Richtung verlaufend ausgerichtet, welche orthogonal zu einer Längsrichtung des Befestigungsabschnitts orientiert ist. Dadurch lässt sich über dieses spitzwinkelig ausgerichtete Federsegment bei Querbelastung des Befestigungsabschnitts oder bei Einleitung einer in Querrichtung verlaufenden Belastung am Befestigungspunkt eine Relativverschiebung des Inselabschnitts zu dem zugehörigen Befestigungsabschnitt herbeiführen, und damit eine Veränderung eines Spalts zwischen den Knochenfragmenten bewirken.

Bei Kombination der beiden vorstehend beschriebenen Varianten können die mehreren an Inselabschnitten ausgestalteten Befestigungspunkte in der Längsrichtung des jeweiligen Befestigungsabschnitts aufeinanderfolgend ausgestaltet sein, wobei benachbart liegende Inselabschnitte identisch zueinander ausgestaltet und punktgespiegelt angeordnet sind, indem die benachbart liegenden Inselabschnitte einander mit den spitzwinkelig verlaufenden Federsegmenten zugewandt liegen. In vorteilhafter Weise können die aufeinanderfolgenden Inselabschnitte hierdurch äußerst kompakt nebeneinander ausgestaltet werden.

Es ist eine weitere Ausgestaltungsmöglichkeit der Erfindung, dass die Befestigungsabschnitte paarweise beidseitig jeweils eines zwischenliegenden Brückenabschnitts liegen, welcher zum Überbrücken eines zwischen den Knochenfragmenten verlaufenden Spalts vorgesehen ist. In Weiterbildung dieser Ausgestaltungsmöglichkeit kann zumindest eines der asymmetrisch angeordneten Federsegmente so ausgerichtet sein, dass bei einer Belastung in einer Hauptbelastungsrichtung des Knochens zwischen dem jeweiligen Inselabschnitt und dem jeweiligen Befestigungsabschnitt eine Relativverschiebung stattfindet, bei welcher sich eine Distanz des jeweiligen Inselabschnitts zu dem Brückenabschnitt vergrößert. Dies hat den Vorteil, dass es somit bei Belastung des Knochens in der Hauptbelastungsrichtung aufgrund der Vergrößerung der Distanz des Inselabschnitts zu dem Brückenabschnitt auch zu einer Vergrößerung des Spalts kommt, welcher mit dem Brückenabschnitt zwischen den Knochensegmenten überbrückt ist. Damit kann vermieden werden, dass der Spalt zwischen den Knochensegmenten zu Null wird und die Knochensegmente aneinander stoßen, was ansonsten den Heilungsprozess des wiederherzustellenden Knochens erschweren würde. Bei einem Einfügen eines resezierten Knochensegments in eine Fehlstelle des wiederherzustellenden Knochens kann hierdurch ebenfalls ein zu kleiner oder nicht vorhandener Spalt zwischen den Knochensegmenten vermieden werden, wenn das resezierte Knochensegment im Hinblick auf die Fehlstelle mit Übergangspassung gestaltet ist.

Im Sinne der Erfindung ist unter der "Belastung in Hauptbelastungsrichtung" des Knochens insbesondere eine Krafteinwirkung auf den Knochen bei dessen üblicher Beanspruchung zu verstehen. Bevorzugt findet diese Belastung dabei derartig statt, dass an dem zugehörigen Befestigungspunkt des Inselabschnitts eine resultierende Querkraft hervorgerufen wird. Im Falle der bevorzugten Ausgestaltung der Osteosyntheseplatte zur Fixierung von Knochenfragmenten zur Wiederherstellung einer Mandibula handelt es sich bei der Belastung in Hauptbelastungsrichtung um eine über die Mandibula abzustützende Beißkraft.

Gemäß einer weiteren Ausgestaltungsmöglichkeit der Erfindung ist bei einem der Befestigungsabschnitte der mindestens eine Befestigungspunkt ortsfest ausgebildet. Bei diesem Befestigungsabschnitt ist also der eine Befestigungspunkt oder sind die mehreren Befestigungspunkte ortsfest ausgestaltet, so dass starre Verbindungen zu dem jeweiligen Knochensegment hergestellt werden können.

Alternativ oder ergänzend dazu sind die Befestigungspunkte bei mehreren Befestigungsabschnitten an Inselabschnitten ausgestaltet. Ist dabei bei keinem der Befestigungsabschnitte eine ortsfeste Ausbildung des mindestens einen Befestigungspunkt vorgenommen, so sind die Befestigungspunkte bei allen Befestigungsabschnitten an Inselabschnitten ausgestaltet.

Vorteilhafte Ausführungsformen der Erfindung, die nachfolgend erläutert werden, sind in den Zeichnungen dargestellt. Es zeigen:
- Fig. 1A und 1B: schematische Darstellungen eines Knochens mit einer hieran befestigten Osteosyntheseplatte gemäß einer Ausführungsform der Erfindung, gezeigt in unterschiedlichen Zuständen; und
- Fig. 2 bis 4: perspektivische Ansichten je einer Osteosyntheseplatte gemäß jeweils einer weiteren Ausführungsform der Erfindung.

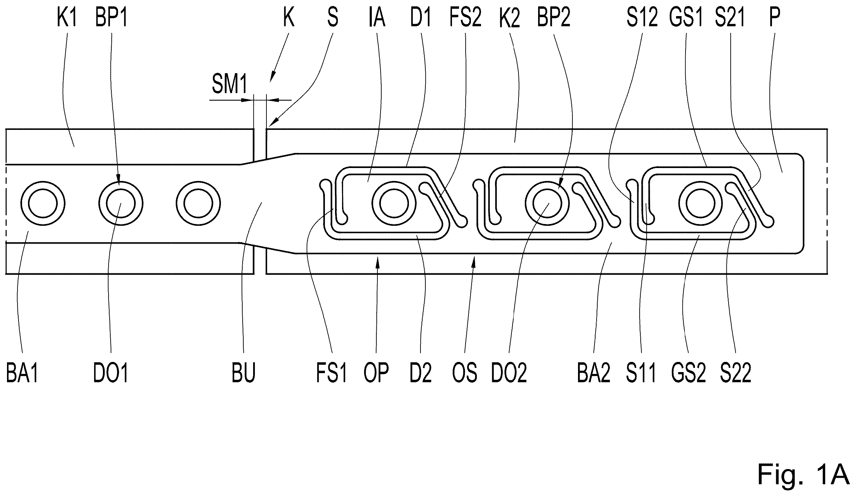

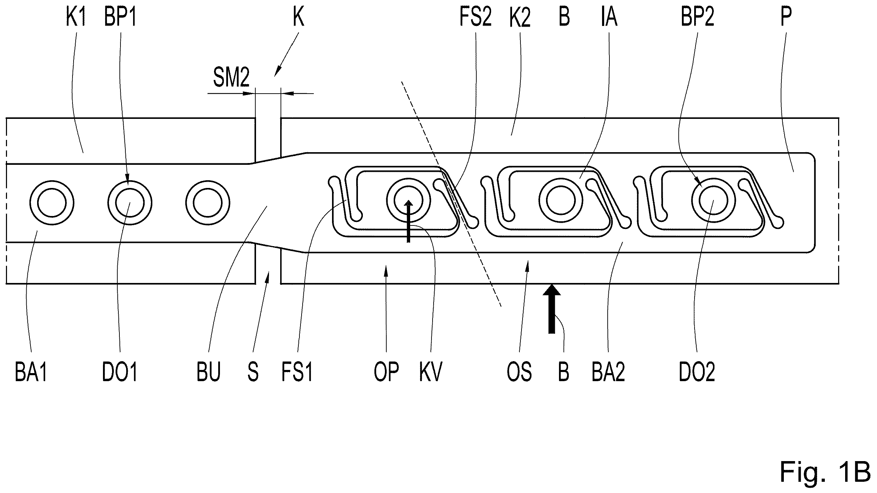

Aus den Fig. 1A und 1B gehen schematische Darstellungen eines Knochens K mit einer hieran befestigten Osteosyntheseplatte OP hervor, wobei es sich bei dem Knochen K hierbei bevorzugt um eine Mandibula handelt. Die Osteosyntheseplatte OP ist an Knochenfragmenten K1 und K2 zu befestigen, um die Knochenfragmente K1 und K2 zueinander zu stabilisieren und hierdurch ein Zusammenwachsen der Knochenfragmente K1 und K2 zur Wiederherstellung des Knochens K zu ermöglichen.

Vorliegend können sich die Knochenfragmente K1 und K2 aufgrund einer Knochenfraktur des Knochens K gebildet haben, wobei die Osteosyntheseplatte OP dann zu einem Ausheilen dieser Knochenfraktur durch die Stabilisierung der Knochenfragmente K1 und K2 zueinander vorgesehen ist. Alternativ dazu kann eines der Knochenfragmente K1 und K2 zum Schließen einer Fehlstelle des Knochens K herangezogen worden sein, beispielsweise weil an dieser Stelle ein Teil des Knochens K aufgrund einer Tumorerkrankung entfernt werden musste. Dabei handelt es sich bei dem einen Knochenfragment K1 bzw. K2 um einen aus einem anderen Knochen, beispielsweise dem Wadenbein, resezierten Knochenteil.

Die Osteosyntheseplatte OP weist einen länglichen Plattenkörper P auf, welcher bevorzugt aus Titan oder einer Titanlegierung besteht und insbesondere in einem additiven Herstellungsprozess, bevorzugt im 3D-Druckverfahren, gefertigt worden ist. Der Plattenkörper P weist eine in den Fig. 1A und 1B sichtbare Oberseite OS und eine gegenüberliegend angeordnete, nicht sichtbare Unterseite auf, auf welcher der Plattenkörper P an den Knochenfragmenten K1 und K2 aufgelegt ist.

Der Plattenkörper P setzt sich aus zwei Befestigungsabschnitten BA1 und BA2 sowie einem, zwischen den Befestigungsabschnitten BA1 und BA2 liegenden Brückenabschnitt BU zusammen. Dabei ist an dem Befestigungsabschnitt BA1 an der Unterseite des Plattenkörpers P eine Befestigung des Plattenkörpers P an dem Knochenfragment K1 vorzunehmen, wobei diese Befestigung dabei an Befestigungspunkten BP1 vorgenommen ist, welche dem Befestigungsabschnitt BA1 zugeordnet sind. Die Befestigungspunkte BP1 sind hierbei als Durchgangsöffnungen DO1 ausgeführt, welche sich jeweils zwischen der Oberseite OS und der Unterseite erstrecken und der Hindurchführung je einer in den Figuren nicht dargestellten Knochenschraube dienen. Dabei sind die Durchgangsöffnungen DO1 in den Befestigungsabschnitt BA1 eingebracht und ortsfest an dem Befestigungsabschnitt BA1 ausgestaltet.

Anschließend an den Befestigungsabschnitt BA1 weist der Plattenkörper P einen Brückenabschnitt BU zur Überbrückung eines Spalts S auf. Der Spalt S liegt zwischen den Knochenfragmenten K1 und K2 vor. Anschließend an den Brückenabschnitt BU ist der Befestigungsabschnitt BA2 ausgebildet, an dem eine Befestigung des Plattenkörpers P seitens des Knochenfragments K2 vorgenommen wird. Dazu sind dem Befestigungsabschnitt BA2 Befestigungspunkte BP2 zugeordnet. Auch die Befestigungspunkte BP2 sind als Durchgangsöffnungen DO2 ausgestaltet, die zwischen der Oberseite OS und der Unterseite verlaufen und für die Hindurchführung je einer in den Figuren nicht dargestellten Knochenschraube vorgesehen sind.

Im Unterschied zu dem Befestigungsabschnitt BA1 sind die Durchgangsöffnungen DO2 bei dem Befestigungsabschnitt BA2 an Inselabschnitten IA eingebracht bzw. definiert, die von dem Befestigungsabschnitt BA2 abgeteilt sind, indem jeder einzelne Inselabschnitt IA mit dem Befestigungsabschnitt BA2 ausschließlich über Federsegmente FS1 und FS2 verbunden ist. Die zugeordneten Federsegmente FS1 und FS2 lassen elastisch federnd Relativverschiebungen des jeweiligen Inselabschnitts IA und damit auch des zugehörigen Befestigungspunktes BP2 zu dem Befestigungsabschnitt BA2 zu.

Die Federsegmente FS1 und FS2 und auch der jeweils zugehörige Inselabschnitt IA sind bei der Osteosyntheseplatte OP über Durchbrüche D1 und D2 im Plattenkörper P definiert, welche U-förmig gestaltet sind und von der Oberseite OS zu der Unterseite verlaufen. Die Durchbrüche D1 und D2 sind dabei mit den Öffnungen ihrer U-Form einander zugewandt ineinander geschachtelt, wodurch neben der Abteilung des jeweiligen Inselabschnitts IA von dem Befestigungsabschnitt BA2 auch die Federsegmente FS1 und FS2 definiert werden. Die U-Formen der Durchbrüche D1 und D2 weichen voneinander ab, indem zwar beide U-Formen je einen ersten, quer zu einer jeweiligen Grundseite GS1, GS2 der U-Form verlaufenden Schenkel S11, S12 aufweisen, bei der U-Form des Durchbruchs D1 dann aber ein zweiter Schenkel S21 gegenüber der Grundseite GS1 nach außen angewinkelt verläuft, wohingegen ein zweiter Schenkel S22 bei der U-Form des Durchbruchs D2 nach innen angewinkelt ist. Hierdurch ergeben sich lineare und asymmetrische Verläufe der Federsegmente FS1 und FS2 zu dem jeweiligen Befestigungspunkt BP2. Aufgrund dieser asymmetrischen Verläufe können die an dem Befestigungsabschnitt BA2 vorgesehenen Inselabschnitte IA gleichgerichtet dicht aufeinanderfolgen.

In Fig. 1A ist ein unbelasteter Zustand des Knochens K gezeigt, wobei die Federsegmente FS1 und FS2 des jeweiligen Inselabschnitts IA eine Grundstellung des jeweiligen Inselabschnitts IA einstellen und sich hierdurch hinsichtlich des Spalts S zwischen den Knochenfragmenten K1 und K2 ein Spaltmaß SM1 ergibt. Dagegen zeigt Fig. 1B den Knochen K bei einer Belastung B, welche in Fig. 1B mit einem Pfeil angedeutet ist und eine übliche Beanspruchung des Knochens K darstellt. So kann es sich bei dieser Belastung B im Falle der bevorzugten Ausführung des Knochens K als Mandibula um eine abzustützende Beißkraft handeln.

Das Federsegment FS2 des jeweiligen Inselabschnitts IA verläuft spitzwinkelig zu einem jeweiligen Kraftvektor KV, durch welchen eine an dem jeweiligen Befestigungspunkt BP2 aufgrund der Belastung B resultierende Querkraft repräsentiert ist und der in Fig. 1B für einen der Befestigungspunkte BP2 mit einem Pfeil angedeutet ist. Zusätzlich zu diesem - in Fig. 1 B gestrichelt angedeuteten - spitzwinkeligen Verlauf zu dem Kraftvektor KV verläuft das Federsegment FS2 des Inselabschnitts IA divergierend zu dem Brückenabschnitt BU. Dies hat zur Folge, dass an dem Federsegment FS2 bei Einleitung der Belastung B in das Knochenfragment K2 eine Kraftumleitung stattfindet, bei welcher die Inselabschnitte IA von dem Brückenabschnitt BU wegbewegt werden und damit eine Distanz zwischen den Inselabschnitten IA und dem Brückenabschnitt BU vergrößert wird. Dies resultiert in einer Auseinanderbewegung der Knochenfragmente K1 und K2 und damit einer Vergrößerung des Spalts S auf ein Spaltmaß SM2. Mit Beendigung der Belastung B verkleinert sich der Spalt S dann wieder auf das Spaltmaß SM1, indem die Federsegmente FS1 und FS2 des jeweiligen Inselabschnitts IA durch elastisches Zurückverformen eine entsprechende Rückverschiebung hervorrufen. Generell wird durch die Relativbewegung der Knochenfragmente K1 und K2 zueinander der Heilungsprozess des Knochens K begünstigt.

Die Spaltmaße SM1, SM2 in Fig. 1A und Fig. 1B sowie die Auslenkung der Inselabschnitte IA in Fig. 1B sind zur besseren Illustration stark übertrieben dargestellt. Bei einer tatsächlichen Anwendung der Osteosyntheseplatte OP, OP' werden selbstverständlich kleinere Spaltmaße verwendet, und eine übliche Belastung B des Knochens K würde in einer geringeren Auslenkung der Inselabschnitte IA resultieren als in Fig. 1B dargestellt.

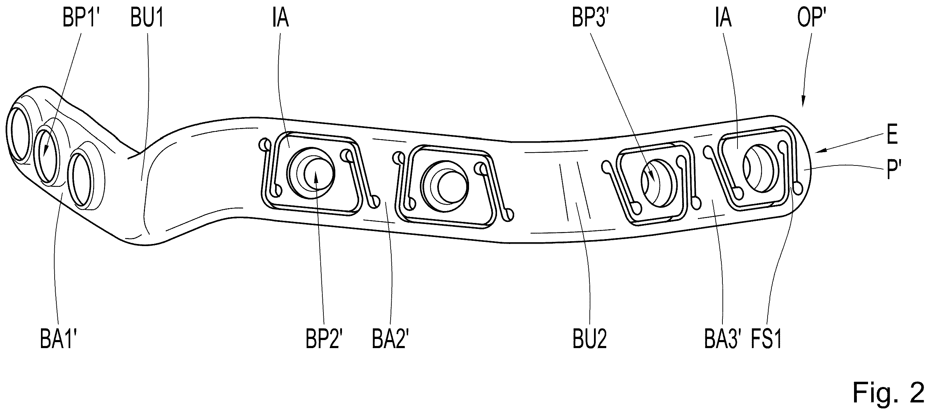

Aus Fig. 2 geht eine perspektivische Darstellung einer Osteosyntheseplatte OP' entsprechend einer weiteren Ausgestaltungsmöglichkeit der Erfindung hervor. Unterschiedlich gegenüber der Variante nach den Fig. 1A und 1B ist hierbei, dass ein Plattenkörper P' dieser Osteosyntheseplatte OP' zwei Brückenabschnitte BU1 und BU2 zum Überbrücken je eines, in Fig. 2 nicht dargestellten Spalts aufweist. Insofern können über die Osteosyntheseplatte OP' drei Knochenfragmente eines Knochens zu dessen Wiederherstellung zueinander stabilisiert werden. Die Brückenabschnitte BU1 und BU2 sind dabei zwischenliegend zu Befestigungsabschnitten BA1', BA2' und BA3' definiert, welche jeweils der Befestigung an ihr einem der Knochenfragmente dienen.

Während Befestigungspunkte BP1' dabei an dem Befestigungsabschnitt BA1' ortsfest ausgestaltet sind, sind die Befestigungspunkte BP2' und BP3' bei den Befestigungsabschnitten BA2' und BA3' jeweils an Inselabschnitten IA definiert, die hierbei in analoger Weise zu der Variante nach den Fig. 1A und 1B gestaltet sind. Bei dem Befestigungsabschnitt BA3' sind die gleichgerichtet angeordneten Inselabschnitte IA dabei so ausgerichtet, dass das Federsegment FS1 des an einem Ende E des Plattenkörpers P' liegenden Inselabschnitts IA diesem Ende E zugewandt liegt. Hierdurch kann dieses zuletzt liegende Inselelement IA dicht an dem Ende E platziert werden. Ansonsten entspricht die Ausgestaltungsmöglichkeit nach Fig. 2 der Variante nach den Fig. 1A und 1B, so dass auf das hierzu Beschriebene Bezug genommen wird.

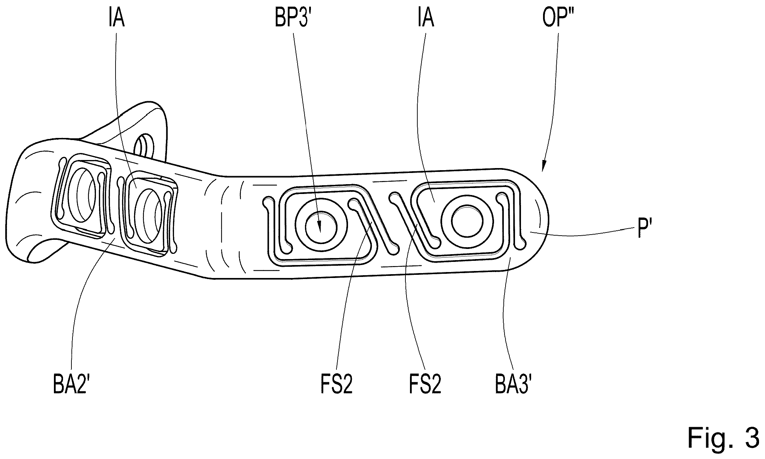

Fig. 3 zeigt eine perspektivische Ansicht einer Osteosyntheseplatte OP" entsprechend einer weiteren Ausgestaltungsmöglichkeit der Erfindung. Diese entspricht dabei weitestgehend der vorhergehenden Variante nach Fig. 2, wobei im Unterschied zu der Osteosyntheseplatte OP' aus Fig. 2 nun sowohl bei dem Befestigungsabschnitt BA2', als auch bei dem Befestigungsabschnitt BA3' die Inselabschnitte IA einander entgegengerichtet angeordnet sind, indem bei den einander entgegen gerichteten Inselabschnitten IA die Federsegmente FS2 einander zugewandt sind. Dies ermöglicht eine im Vergleich zu der Variante nach Fig. 2 noch kompaktere Ausgestaltung der nebeneinanderliegenden Inselabschnitte IA bei dem jeweiligen Befestigungsabschnitt BA2' bzw. BA3'. Im Übrigen entspricht die Ausgestaltungsmöglichkeit nach Fig. 3 der Variante nach Fig. 2, so dass auf das hierzu Beschriebene Bezug genommen wird.

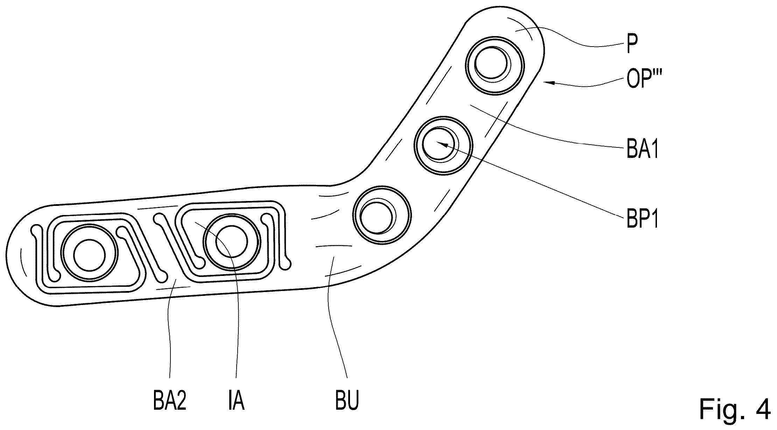

Außerdem geht noch aus Fig. 4 eine perspektivische Darstellung einer Osteosyntheseplatte OP‴ hervor, welche entsprechend einer weiteren Ausführungsform der Erfindung ausgebildet ist und im Wesentlichen der vorhergehenden Variante nach Fig. 3 entspricht. Unterschiedlich ist dabei, dass ein Plattenkörper P dieser Osteosyntheseplatte OP‴ nun lediglich einen Brückenabschnitt BU sowie zwei beidseitig hierzu liegende Befestigungsabschnitte BA1 und BA2 aufweist. Während Befestigungspunkte BP1 bei dem Befestigungsabschnitt BA1 ortsfest ausgestaltet sind, sind die dem Befestigungsabschnitt BA2 zugeordneten Befestigungspunkte BP2 an Inselabschnitten IA ausgebildet. In Übereinstimmung mit der vorhergehenden Variante nach Fig. 3 sind diese Inselabschnitte IA einander entgegen gerichtet, indem die Federsegmente FS2 dieser Inselabschnitte IA einander zugewandt sind. Auch ansonsten entspricht die Ausführungsform nach Fig. 4 der Variante nach Fig. 3, so dass auf das hierzu Beschriebene Bezug genommen wird.

Mittels der erfindungsgemäßen Ausgestaltungen kann jeweils eine Osteosyntheseplatte geschaffen werden, bei welcher eine kompakte Anordnung von über Federsegmente relativ verschiebbaren Befestigungspunkten verwirklicht ist.

### Bezugszeichenliste

- K: Knochen
- OP, OP', OP", OP‴: Osteosyntheseplatte
- K1, K2: Knochenfragmente
- P, P': Plattenkörper
- OS: Oberseite
- BA1, BA2, BA1', BA2', BA3': Befestigungsabschnitte
- BU, BU1, BU2: Brückenabschnitt
- BP1, BP2, BP1', BP2', BP3': Befestigungspunkte
- DO1, DO2: Durchgangsöffnungen
- S: Spalt
- IA: Inselabschnitte
- FS1, FS2: Federsegmente
- D1, D2: Durchbrüche
- GS1, GS2: Grundseiten
- S11, S12, S21, S22: Schenkel
- SM1, SM2: Spaltmaße
- B: Belastung
- KV: Kraftvektor
- E: Ende

## Patentansprüche

1. Osteosyntheseplatte (OP; OP'; OP"; OP‴), welche für eine Fixierung von Knochenfragmenten (K1, K2) zur Wiederherstellung eines Knochens (K), insbesondere einer Mandibula, vorgesehen ist, umfassend einen Plattenkörper (P; P') mit einer Oberseite (OS) und einer Unterseite, auf welcher der Plattenkörper (P; P') an den Knochenfragmenten (K1, K2) aufzulegen ist, wobei der Plattenkörper (P; P') mehrere Befestigungsabschnitte (BA1, BA2; BA1', BA2', BA3') aufweist, an welchen auf der Unterseite Befestigungen der Osteosyntheseplatte (OP; OP'; OP"; OP‴) seitens der Knochenfragmente (K1, K2) vorzunehmen sind und denen dazu jeweils mindestens ein Befestigungspunkt (BP1, BP2; BP1', BP2', BP3') zugeordnet ist, wobei der mindestens eine Befestigungspunkt (BP2; BP2', BP3') bei zumindest einem der Befestigungsabschnitte (BA1, BA2; BA1', BA2', BA3') an einem Inselabschnitt (IA) ausgestaltet ist, welcher mit dem zugehörigen Befestigungsabschnitt (BA2; BA2', BA3') des Plattenkörpers (P; P') ausschließlich über Federsegmente (FS1, FS2) verbunden ist, welche eine Relativverschiebung des jeweiligen Befestigungsabschnitts (BA2; BA2', BA3') und des jeweiligen Inselabschnitts (IA) zueinander elastisch federnd zulassen, **dadurch gekennzeichnet, dass** die Federsegmente (FS1, FS2) den ihnen zugeordneten Befestigungspunkt (BP2, BP2', BP3') asymmetrisch umgeben.

2. Osteosyntheseplatte (OP; OP'; OP"; OP‴) nach Anspruch 1, **dadurch gekennzeichnet, dass** der Inselabschnitt (IA) über genau zwei Federsegmente (FS1, FS2) mit dem jeweiligen Befestigungsabschnitt (BA2; BA2', BA3') verbunden ist.

3. Osteosyntheseplatte (OP; OP'; OP"; OP‴) nach Anspruch 1 oder 2, **dadurch gekennzeichnet, dass** der Inselabschnitt (IA) und die zugeordneten Federsegmente (FS1, FS2) durch an dem jeweiligen Befestigungsabschnitt (BA2; BA2', BA3') ausgestaltete Durchbrüche (D1, D2) gebildet sind.

4. Osteosyntheseplatte (OP; OP'; OP"; OP‴) nach Anspruch 3, **dadurch gekennzeichnet, dass** die Durchbrüche (D1, D2) U-förmig ausgestaltet sind, wobei die U-förmigen Durchbrüche (D1, D2) mit ihren Öffnungen einander zugewandt und ineinander geschachtelt ausgestaltet sind.

5. Osteosyntheseplatte (OP; OP'; OP"; OP‴) nach wenigstens einem der vorhergehenden Ansprüche, **dadurch gekennzeichnet, dass** ein erstes der Federsegmente (FS1, FS2) orthogonal zu einer Längsrichtung des Befestigungsabschnitts (BA2; BA2', BA3') orientiert ist.

6. Osteosyntheseplatte (OP; OP'; OP"; OP‴) nach wenigstens einem der vorhergehenden Ansprüche, **dadurch gekennzeichnet, dass** dem jeweiligen Befestigungsabschnitt (BA2; BA2', BA3') mehrere Befestigungspunkte (BP2; BP2', BP3') zugeordnet sind, wobei jeder der Befestigungspunkte (BP2; BP2', BP3') an jeweils einem Inselabschnitt (IA) mit asymmetrisch um den jeweiligen Befestigungspunkt (BP2; BP2', BP3') angeordneten Federsegmenten (FS1, FS2) ausgestaltet sind.

7. Osteosyntheseplatte (OP; OP'; OP"; OP‴) nach wenigstens einem der vorhergehenden Ansprüche, **dadurch gekennzeichnet, dass** ein zweites der Federsegmente (FS1, FS2) spitzwinkelig zu einer Richtung verlaufend ausgerichtet ist, welche orthogonal zu einer Längsrichtung des Befestigungsabschnitts (BA2; BA2', BA3') orientiert ist.

8. Osteosyntheseplatte (OP"; OP"') nach den Ansprüchen 6 und 7, **dadurch gekennzeichnet, dass** die mehreren an Inselabschnitten (IA) ausgestalteten Befestigungspunkte (BP2; BP2', BP3') in der Längsrichtung des jeweiligen Befestigungsabschnitts (BA2; BA2', BA3') aufeinanderfolgend ausgestaltet sind, wobei benachbart liegende Inselabschnitte (IA) identisch zueinander ausgestaltet und punktgespiegelt angeordnet sind, indem die benachbart liegenden Inselabschnitte (IA) einander mit den spitzwinkelig verlaufenden Federsegmenten (FS2) zugewandt liegen.

9. Osteosyntheseplatte (OP; OP'; OP"; OP‴) nach wenigstens einem der vorhergehenden Ansprüche, **dadurch gekennzeichnet, dass** die Befestigungsabschnitte (BA1, BA2; BA1', BA2', BA3') paarweise beidseitig jeweils eines zwischenliegenden Brückenabschnitts (BU; BU1, BU2) liegen, welcher zum Überbrücken eines zwischen den Knochenfragmenten (K1, K2) verlaufenden Spalts (S) vorgesehen ist.

10. Osteosyntheseplatte (OP; OP') nach Anspruch 9, **dadurch gekennzeichnet, dass** zumindest eines der asymmetrisch angeordneten Federsegmente (FS1, FS2) so ausgerichtet ist, dass bei einer Belastung (B) in einer Hauptbelastungsrichtung des Knochens (K) zwischen dem jeweiligen Inselabschnitt (IA) und dem jeweiligen Befestigungsabschnitt (BA2; BA2') eine Relativverschiebung stattfindet, bei welcher sich eine Distanz des jeweiligen Inselabschnitts (IA) zu dem Brückenabschnitt (BU; BU1) vergrößert.

11. Osteosyntheseplatte (OP; OP'; OP"; OP‴) nach wenigstens einem der vorhergehenden Ansprüche, **dadurch gekennzeichnet, dass** bei einem der Befestigungsabschnitte (BA1, BA2; BA1', BA2', BA3') der mindestens eine Befestigungspunkt (BP1; BP1') ortsfest ausgebildet ist.

12. Osteosyntheseplatte (OP'; OP") nach wenigstens einem der vorhergehenden Ansprüche, **dadurch gekennzeichnet, dass** die Befestigungspunkte (BP2', BP3') bei mehreren oder allen Befestigungsabschnitten (BA2', BA3') an Inselabschnitten (IA) ausgestaltet sind.
